# EUROPEAN PATENT SPECIFICATION

(11) **EP 3 774 951 B1**
(45) Date of publication and mention of the grant of the patent: **31.01.2024**
(21) Application number: 19719430.1
(22) Date of filing: 29.03.2019
(51) Int. Cl.: C08F 230/06, C08F 2/48, A61B 5/145, G01N 33/50, G01N 33/66

(54) **BORONIC ACID DERIVATIVES FOR DIOL-SENSING HYDROGELS**
BORONSÄUREDERIVATE FÜR DIOL-ERFASSENDE HYDROGELE
DÉRIVÉS D'ACIDE BORONIQUE POUR HYDROGELS DE DÉTECTION DE DIOLS

(30) Priority: 29.03.2018 GB 201805226
(43) Date of publication of application: 17.02.2021
(73) Proprietor: The Provost, Fellows, Scholars and other Members of Board of Trinity College Dublin, Dublin 2 (IE)
(72) Inventor: DELANEY, Colm, Dublin, Dublin 2 (IE); FLOREA, Larisa, Elena, Dublin, Dublin 2 (IE); BRUEN, Danielle, Dublin, 13 (IE); DIAMOND, Dermot, Dublin (IE)
(74) Representative: Purdylucey Intellectual Property
(86) International application number: PCT/EP2019/058061
(87) International publication number: WO 2019/185897

(56) References cited:
- WO-A1-2009/087373
- WO-A1-2011/018752
- WO-A1-2017/063131
- WO-A2-2016/154317
- CN-A- 107 474 184
- US-B2- 7 470 420
- US-B2- 7 718 804
- ZACHARY SHARRETT ET AL: "Exploring the use of APTS as a fluorescent reporter dye for continuous glucose sensing", ORGANIC & BIOMOLECULAR CHEMISTRY, vol. 7, no. 7, 1 January 2009 (2009-01-01) , page 1461, XP055594647, ISSN: 1477-0520, DOI: 10.1039/b821934f

## Description

### Field of the Invention

The present invention relates to a family of polymerisable boronic-acids, diol-sensing hydrogels, and a sensor device comprising a diol-sensing hydrogel. Also contemplated are methods of making the hydrogel, methods of use of the hydrogel and sensor in the qualitative and quantitative sensing of diols, including sugar and lactate.

### Background to the invention

A number of polymerisable boronic acids currently exist in the literature and as commercially available materials. For the most part, these exist as short chain, polymerisable monomers based on a substituted phenyl boronic acid. Hydrogel materials based on these boronic acids, copolymerised with other monomers (e.g. acrylamide) are widely used in gels which respond to saccharide binding by an increase in size and weight. While useful properties, these traits do not lend themselves to a feasible real-time sensing technology. Indeed, the number of optically responsive boronic acid hydrogel materials are really very few. Some attempts in the literature apply models proven in solution studies to the gel matrix, but no constructive outcomes exist in the market place. In other research, it has been necessary to incorporate another chromophore/fluorophore into the gel structure, which can modulate its colorimetric/fluorescent response based on the binding of a saccharide to a boronic acid in the local environment of the chromophore/fluorophore.

US7718804 and US7470420 disclose quaternary nitrogen heterocyclic boronic acid containing compounds for detecting monosaccharides by fluorescence. The heterocyclic boronic acid is designed to incorporate within its structure (US7718804) or interact with an external fluorophore (US7470420), and sensing of sugar is based on fluorescence spectroscopy.

### Summary of the Invention

The present invention provides a class of quaternised ammonium boronic monomers, their easily adaptable synthesis, and polymerisation to yield diol-sensing hydrogels, especially 1,2- or 1,3-diols such as sugars, catechols, lactic acids (and lactate), sialic acids, α-hydroxy carboxylates, glucosamine, ribose, and adenosine triphosphate (ATP), among others. The hydrogels comprise a crosslinked polymeric matrix formed from a polymerizable boronic acid monomer that is capable of exhibiting a non-fluorescent optical response in the presence of a diol, which is sufficiently sensitive to detect the levels of diols found in biological fluids such as sweat and ocular fluid. The advantage over the fluorescent boronic acid probes of US7718804 and US7470420 is that the boronic acid moiety does not need to incorporate a fluorophore, thereby providing greater flexibility of design. In addition, the hydrogels of the present invention exhibit changes in opacity/transparency, which is easier to detect and quantify than fluorescence.

In a first aspect, there is provided a hydrogel comprising a crosslinked polymeric matrix formed from a polymerizable boronic acid salt monomer having the general structure (I):

A+.X- (I)

in which:
A represents a quaternised ammonium boronic acid cation; and
X represents an anion,
wherein either A or X contains a free-radical polymerizable group.

Typically, the hydrogel is a sugar sensing hydrogel.

In another aspect, there is provided a sensor device, the sensor device comprising a device body and a hydrogel according to the invention associated with the device body.

In another aspect, there is provided a polymerizable boronic acid salt having the general structure (I):

A+.X- (I)

in which:
A represents a quaternised ammonium boronic acid cation; and
X represents an anion,
wherein either A or X contains a free-radical polymerizable group.

In another aspect, there is provided a method of making a hydrogel according to the invention, the method comprising the steps of:
(a) pre-mixing a polymerizable boronic acid according to the invention and a cross-linking agent;
(b) dissolving the pre-mixture of step (a) in water, an organic solvent or a mixture of solvents;
(c) adding a radical polymerisation initiator to the pre-mixture to form a mixture; and
(d) polymerizing the mixture.

In another aspect, the invention provides a hydrogel formed according to a method of the invention.

In another aspect, the invention provides a curable composition comprising a polymerizable boronic acid according to the invention, a cross-linking agent, and a radical polymerisation initiator.

In one embodiment, the boronic acid is non-fluorescent.

In one embodiment, the boronic acid is a phenyl boronic acid.

In one embodiment, the polymeric matrix comprises greater than 20%, 30%, 40%, 50%, 60%, 70%, 80%, or 90% polymerizable boronic acid salt monomer.

In one embodiment, the free-radical polymerizable group is selected from a (meth)acrylamide, (meth)acrylate, styrene, vinyl ether or vinyl group.

In one embodiment, the polymerizable boronic acid salt has a general structure (II): 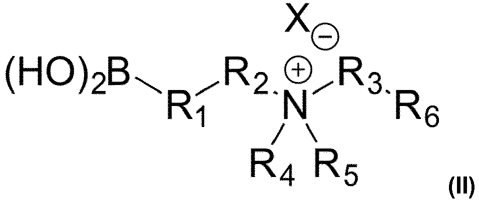 in which:
(OH)₂B-R1 is a boronic acid;
R2 to R5 are each, independently, an aryl or alkyl group; and
R6 is alkyl, aryl, hydroxyl, or a free-radical polymerizable group.

In one embodiment, the polymerizable boronic acid salt has a general structure (III): 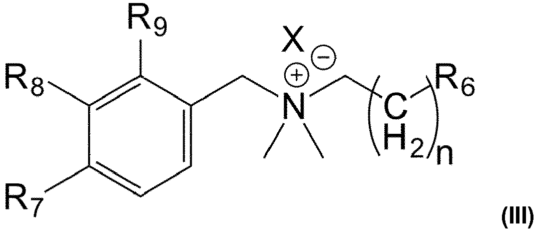 in which:
R7 to R9 are each, independently, selected from B(OH)₂, H;
n is a whole number selected from 0-6;
R6 is alkyl, aryl, hydroxyl, or a free-radical polymerizable group; and
one of R7 to R9 is B(OH)₂.

In one embodiment, (OH)₂-R1 is a non-heterocyclic boronic acid. In one embodiment, (OH)₂-R1 is a phenyl boronic acid.

In one embodiment, one of R7 to R9 is B(OH)₂, and the other two of R7 to R9 is H.

In one embodiment, R9 is B(OH)₂, and R7 and R8 is H. In another embodiment, R8 is B(OH)₂, and R7 and R9 is H. In another embodiment, R7 is B(OH)₂, and R9 and R8 is H.

In one embodiment, the cation A contains the free-radical polymerizable group. In this embodiment, the cation includes R6 (free-radical polymerizable group), and the anion is typically selected from a halide, sulphonate, sulphinate, phosphonate, phosphinate, and carboxylate.

In another embodiment, the anion X contains the free-radical polymerizable group. In this embodiment, the anion X has a general structure (IV):

Y-(CH₂)ₘ-Z (IV)

in which:
m is a whole number selected from 0-6;
Y is a substituent selected from a sulphonate, sulphinate, phosphonate, phosphinate, and carboxylate; and
Z is a substituent selected from (meth)acrylamide, (meth)acrylate, styrene, vinyl ether or vinyl group.

Thus, in one embodiment, the polymerizable boronic acid salt has the general structure (V): 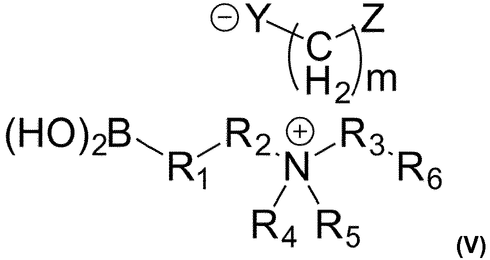 in which:
R1 to R5, Y and m are as defined above;
R6 is alkyl, aryl or hydroxyl; and
Z is a free-radical polymerizable group.

In another embodiment, the polymerizable boronic acid salt has the general structure (VI): 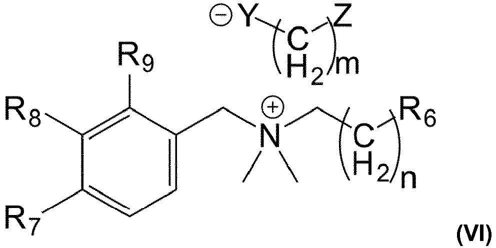 in which:
R7 to R9, Y, m, and n, are as defined above;
R6 is alkyl, aryl or hydroxyl; and
Z is a free-radical polymerizable group.

In one embodiment, the free-radical polymerizable group is selected from a (meth)acrylamide, (meth)acrylate, styrene, vinyl ether or vinyl group.

In one embodiment, the free-radical polymerizable group is selected from the following groups:

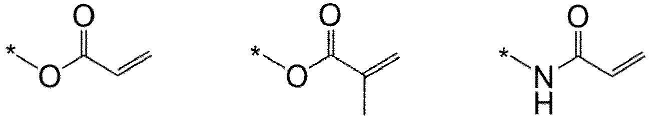

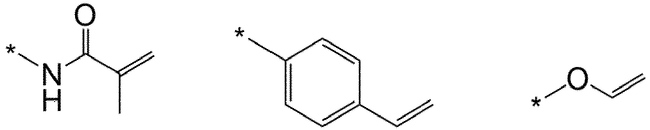

In one embodiment, the polymer is a homo-polymer formed from the polymerizable boronic acid salt monomer of the invention.

In another embodiment, the polymer matrix is a co-polymer formed from the polymerizable boronic acid salt monomer of the invention and a different monomer, for example a different acrylated monomer.

In one embodiment, the hydrogel is in the form of a film.

In one embodiment, the hydrogel is configured to exhibit reduced opacity on binding a diol, especially 1,2- or 1,3- diol.

In one embodiment, the hydrogel is configured to provide a gravimetric response on binding diol.

The invention also provides a sensor device comprising a hydrogel of the invention disposed on a device body.

In one embodiment, the device body is selected from a plaster, patch, bandage, strap, or contact lens.

In one embodiment, the device body is a microfluidic chip having at least one microfluidic channel defining a fluid path and having an opening for receipt of a test fluid and a sensing zone comprising the hydrogel in fluid communication with the fluid path.

In one embodiment, the microfluidic chip comprises a viewing window configured to allow visual monitoring of the hydrogel in the sensing zone.

In one embodiment, the microfluidic chip comprises a detector configured to detect changes in opacity of the hydrogel by electrochemical, impedance, absorbance, or fluorescence spectroscopy, or gravimetric analysis.

Also provided is a contact lens incorporating a polymer formed from a polymerizable boronic acid salt monomer of the invention.

Also provided is a method of making a hydrogel according to the invention.

Also provided is a curable composition comprising a polymerizable boronic acid according to invention, a cross-linking agent, and a radical polymerisation initiator.

In one embodiment, the radical polymerisation initiator is a UV light initiator, and in which the method includes a step of exposing the mixture to UV light.

In one embodiment, the cross-linking agent comprises at least two polymerizable groups and has a molecular weight of at least 100 g.mole⁻¹.

In one embodiment, the composition or mixture includes another acrylated monomer.

Also provided is a method of qualitatively or quantitatively detecting diol, especially 1,2- or 1,3- diol, in an environment comprising a step of placing a hydrogel according to the invention in the environment and monitoring an optical response of the hydrogel in the environment.

The environment may be a sample such as a biological fluid such as saliva, sweat or ocular fluid.

In one embodiment, the optical response is a change in transparency or opacity of the hydrogel.

In one embodiment, the environment is mammalian tissue, for example an ocular surface or the skin of a mammal.

Other aspects and preferred embodiments of the invention are defined and described in the other claims set out below.

### Brief Description of the Figures

**Figure 1****:** Change in opacity of a para-DMAPBA hydrogel film; (A) after hydration in pH 7.4 PBS buffer; (B) After contact with 100 mM Fructose droplets (in pH 7.4 PBS Buffer).
**Figure 2****:** Reversible change in opacity/transparency of a para-DMAPBA hydrogel film when immersed in glucose solution (100 mM) and PBS, respectively. From left to right: after partial immersion in a 100 mM glucose solution the film was immersed in PBS, thereby gradually regaining the opacity of the film over a 10 min period.
**Figure 3****:** Images showing the Y-shaped microfluidic device channel containing the 80 µm thin DMAPBA hydrogel film; (A) The hydrogel film turns opaque as pH 7.4 PBS Buffer is passed through the microfluidic channel; (B) The hydrogel film becomes transparent, revealing the text placed behind ("Insight"), upon passing a glucose solution (100 mM) through the microfluidic channel for 1 min.
**Figure 4****:** Real-time recording of absorbance at 750 nm of the micro-fluidic integrated para-DMAPBA hydrogel film when 100 mM glucose and PBS solutions, respectively, are passed through the microchannel at a flow rate of 200 µL min⁻¹. 100 mM glucose is introduced at min 2 and min 120, respectively; PBS solution is introduced through the channel at min 30 and min 145, respectively. The small spikes observed in the graph indicate the presence of air bubbles.
**Figure 5****:** Real-time recording of absorbance at 750 nm of the micro-fluidic integrated para-DMAPBA hydrogel film with varying concentrations of glucose added (from 1 mM-100 mM), indicated by annotations, at a flowrate of 50 µL min⁻¹.
**Figure 6****:** Analysis of hydrogel absorbance at 750 nm, over time, for varying glucose concentrations. Inset shows normalised change in absorbance for each concentration after 350s, at a flowrate of 50 µL min⁻¹.
**Figure 7.** A) Analysis of hydrogel absorbance at 750 nm over time, when exposed to varying lactate concentrations (10, 25, 50, and 100 mM sodium lactate in PBS buffer, respectively). B: Inset shows normalised change in absorbance of the hydrogel disks for each concentration after 120s.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

In this specification, the term "sugar-sensing hydrogel" refers to matrix formed of crosslinked polymer chains in an aqueous medium, in which at least some of the polymer chains are formed from polymerizable boronic acid salt monomers capable of exhibiting altered optical properties in respond to binding of sugar. In one embodiment, the sugar-sensing hydrogel is non-fluorescent.

In this specification, the term "diol" refers to a chemical compound containing two hydroxy groups, and in particular refers to 1,2- or 1,3- diols, examples of which include sugars, catechols, lactic acids (and lactate), sialic acids, α-hydroxy carboxylates, glucosamine, ribose, and adenosine triphosphate (ATP), among others.

As used herein, the term "crosslinked" as applied to the polymer chains of the hydrogel means that the polymer chains are covalently crosslinked with a crosslinking agent (moiety) to form a three-dimensional network. Examples of crosslinking agents include N,N'-methylenebisacrylamide N,N'-ethylenebisacrylamide, butanediol diacrylate, hexanedioldiacrylate, poly(ethyleneglycol) diacrylate and poly(propyleneglycol) diacrylate.

In this specification, the term "polymeric matrix" refers to the 3-D dimensional network formed by the cross-linked polymer chains.

As used herein, the term "anion" refers to an atom or radical having a negative charge that is capable of combining with the cation to provide a salt. Examples of anions that may be employed in the present invention are halides (chloride, bromide, fluoride, astanide and iodide), sulphates (i.e. alkyl sulphates), sulphonates (i.e. aryl sulphonates), sulphinates, phosphonates (salt or ester of phosphonic acid), phosphinates (i.e. hypophosphite), and carboxylates (salt or ester of carboxylic acid). In one embodiment, the anion contains a free-radical polymerizable group. In such cases, the anion generally has a structure Y-(CH₂)ₘ-Z, in which Y, Z and m are as defined above.

As used herein, the term "quaternised ammonium boronic acid cation" refers to a compound containing a boronic acid covalently linked to a quaternary ammonium cation, and optionally containing a free-radical polymerizable group covalently bonded to the quaternary ammonium cation, which has a positive charge and that is capable of combining with the anion to provide a salt. The boronic acid has the general structure R-B(OH)₂ in which R is a substituent such as phenyl, methyl and propene. Examples of boronic acids useful in the present invention include phenyl boronic acids, for example phenylboronic acid, 2-thienylboronic acid, methylboronic acid, cis-propenylboronic acid, and trans-propenylboronic acid. The quaternary ammonium cation has a general structure NR₄⁺ in which the R are the same or different and are each, independently, selected from an alkyl or an aryl group. In one embodiment, the boronic acid does not incorporate a fluorophore.

As used herein, the term "polymerizable boronic acid salt monomer" refers to a salt comprising an anion and a quaternised ammonium boronic acid cation, in which one of the cation or anion includes a free-radical polymerizable group that allows free radical polymerisation of the salt monomer, optionally in combination with a second monomer.

As used herein, the term "free-radical polymerizable group" refers to a group polymerisable by chain-growth polymerisation which involves the successive additional of free-radical building blocks started using a free-radical initiator.

Examples of free-radical polymerisable groups include (meth)acrylamide, (meth)acrylate, vinyl ether, styrene, or vinyl groups. Specific examples are provided in structures VII-XII above.

As used herein, the term "Alkyl" refers to a group containing from 1 to 8 carbon atoms and may be straight chained or branched. An alkyl group is an optionally substituted straight, branched or cyclic saturated hydrocarbon group. When substituted, alkyl groups may be substituted with up to four substituent groups, at any available point of attachment. When the alkyl group is said to be substituted with an alkyl group, this is used interchangeably with "branched alkyl group". Exemplary unsubstituted groups include methyl, ethyl, propyl, isopropyl, α-butyl, isobutyl, pentyl, hexyl, isohexyl, 4, 4-dimethylpentyl, octyl, 2,2,4-trimethylpentyl, nonyl, decyl, undecyl, dodecyl, and the like. Exemplary substituents may include but are not limited to one or more of the following groups: halo (such as F, Cl, Br, I), Haloalkyl (such as CCl₃ or CF₃), alkoxy, alkylthio, hydroxyl, carboxy (-COOH), alkyloxycarbonyl (-C(O)R), alkylcarbonyloxy (-OCOR), amino (-NH₂), carbamoyl (-NHCOOR-or-OCONHR), urea (-NHCONHR-) or thiol (-SH). Alkyl groups as defined may also comprise one or more carbon double bonds or one or more carbon to carbon triple bonds.

As used herein, the terms "alkyl", "cycloalkyl", "heterocycloalkyl", "cycloalkylalkyl", "aryl", "acyl", "aromatic polycycle", "heteroaryl", "arylalkyl", "heteroarylalkyl", "amino acyl", "non-aromatic polycycle", "mixed aryl and non-aryl polycycle", "polyheteroaryl", "non-aromatic polyheterocyclic", "mixed aryl and non-aryl polyheterocycles", "amino", and "sulphonyl" are defined in US6,552,065, Column 4, line 52 to Column 7, line 39.

As used herein, the term "homo-polymer" as applied to the hydrogel of the invention refers to a polymer that is formed from one type of monomer. The term "co-polymer" refers to a polymer formed from different monomers, for example the polymerizable boronic acid salt of the invention and one or more additional monomers, for example an acrylated monomer.

As used herein, the term "gravimetric response" refers to a change in weight of the hydrogel.

As used herein, the terms "sugar" or "saccharide" are used interchangeably and refer to glucose, fructose, monosaccharides, disaccharides or polysaccharides.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### Materials and reagents

2-(bromomethyl)phenylboronic acid, 3-(bromomethyl)phenylboronic acid, 4-(bromomethyl)-phenylboronic acid, *N*-[3-(Dimethylamino)propyl]methacrylamide, 3-Dimethylamino-1-propanol, 3-(Dimethylamino)propyl acrylate, 2-(Dimethylamino)ethyl methacrylate, potassium 3-sulfopropyl acrylate (KSPA), polyethylene glycol diacrylate (M_{w}~ 320, 100 ppm MEHQ as inhibitor) (PEG256), *N,N'*-Methylenebis(acrylamide (MBIS), 2-Hydroxy-2-methyl-propiophenone 97% (HMPP), Pentaerythritol triacrylate, 7-Diethylamino-3-thenoylcoumarin, tetrabutylphosphonium chloride, ethanol (Chromasolv^{®}, HPLC grade, ≥99.8%), dichloromethane, and diethylether were purchased from Sigma Aldrich^{®} and used as received. Deionized water (18.2 MΩ·cm⁻¹) (DI water) was purified using a Merck Millipore Milli-Q Water Purification System.

### Synthesis of N-(2-boronobenzyl)-3-hydroxy-N,N-dimethylpropan-1-ammonium bromide (ChBA)

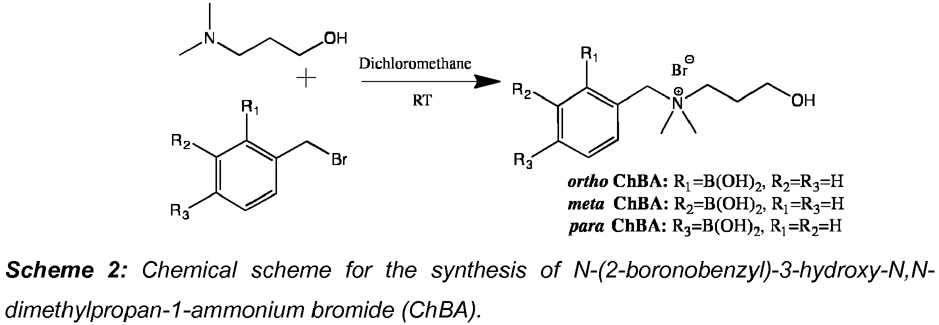

2-(bromomethyl)phenylboronic acid (1 g, 4.65 mmol) was dissolved in dichloromethane (50 mL). To this, 3-dimethylamino-1-propanol (2 mL) was added dropwise and the solution stirred overnight (18h). The solvent was then removed *in vacuo.* The product was isolated as a thick viscous colourless liquid.

### Synthesis of N-(2-boronobenzyl)-3-hydroxy-N,N-dimethylpropan-1-ammonium 3-(acryloyloxy)-propane-1-sulfonate (ChSPABA)

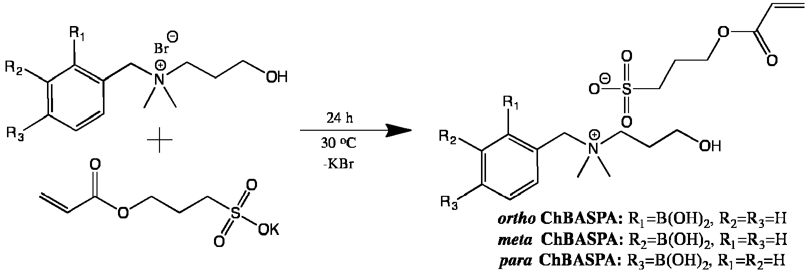

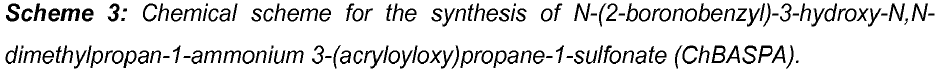

The metathesis reaction, to form the polymerisable ionic liquid was carried out following a modified procedure from Tudor *et al.⁹* ChBA (1.67 g, 3.87 mmol) and potassium 3-sulfopropyl acrylate (1.14 g, 5 mmol) were dissolved in deionised water (25 mL) and stirred for 24h at 30 °C. After this time, the solvent was removed by evaporation. The product was then dissolved in absolute ethanol, the solution and the solvent evaporated using on a rotary evaporator. Then product was dried under vacuum on a high vacuum line and was collected as a thick, viscous oil.

### Generic synthesis of N-(boronobenzyl)methacrylamido-N,N-dimethylpropan-1-ammonium bromide (DMAPBA)

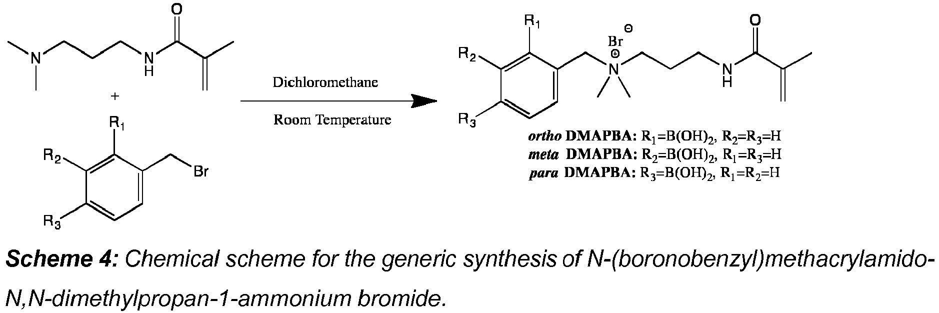

(Bromomethyl)phenyboronic acid (1 g, 4.6 mmol) was dissolved in 25 mL of diethylether and stirred at room temperature. *N*-[3-(dimethylamino)propyl]methacrylamide (2 mL, 10.5 mmol) was added dropwise to the solution. The reaction was stirred overnight (18h). After this time, the solvent was decanted of.. Any remaining solvent was removed *in vacuo.* The product was isolated without further purification as a thick, viscous, colourless liquid.

### Generic synthesis of 3-(acryloyloxy)-N-(2-boronobenzyl)-N,N-dimethylpropan-1-ammonium bromide

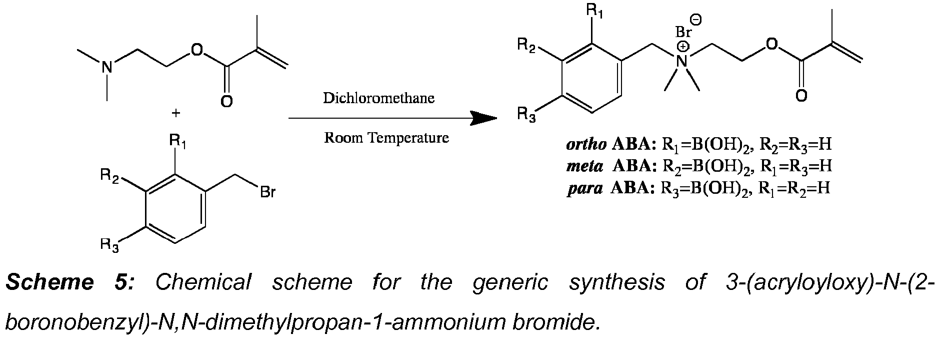

4-(bromomethylphenyl)boronic acid (1.00 g, 4.6 mmol) was stirred at room temperature in dichloromethane (30 mL) to dissolve. 2-(Dimethylamino)ethyl methacrylate (0.86 mL, 5.1 mmol) was added dropwise to the BA while stirring. The reaction was stirred under N₂ for 24h. Diethyl ether (50 mL) was added to precipitate the product as a white crystalline powder.

### Hydrogel Synthesis

A series of soft hydrogels were fabricated from the monomers presented herein, using various length crosslinkers (e.g. PEG 256, MBIS) and a range of white-light and UV initiators. The hydrogels could be made using 100% of the BA monomer, or by copolymerization with other monomers. The gels were polymerized in soft circular polydimethylsiloxane (PDMS) moulds using a UV irradiation (UVP CL-1000 Ultraviolet Crosslinker curing chamber wavelength: 365 nm; power level: 3.5 mW·cm⁻²) for 30 minutes, or through a photomask. An example of a homopolymer hydrogel recipe is show in Table 1.

**Table 1. Example of recipe used for the production of glucose-responsive hydrogels.**

| Material | M_{w} (g/mol) | Weight (mg) | Moles (mol) | Density (g/ml) | Volume (µL) | Molar percent (%) |
|---|---|---|---|---|---|---|
| *Para-*DMAPBA | 411.15 | 270 | 6.567 x 10⁻⁴ | - | - | 100 |
| MBIS | 154.17 | 1 | 6.567 x 10⁻⁶ | | | 1 |
| HMPP | 164.20 | 1 | 6.567 x 10⁻⁶ | 1.077 | 1 | 1 |
| Solvent | | | | | | |
| DMSO | - | - | - | - | 125 | - |
| DI water | - | - | - | - | 125 | - |

### Example of Optical Response

Hydrogel thin films were prepared in a home-made cell consisting of a glass slide and a poly(methyl methacrylate) (PMMA) transparent cover separated by a 80 µm high spacer made from pressure sensitive adhesive (PSA). The cell was filled by capillary action with the monomer solution and subsequently exposed to UV light (UVP CL-1000 Ultraviolet Crosslinker curing chamber wavelength: 365 nm; power level: 3.5 mW·cm⁻²) with or without a photo-mask.

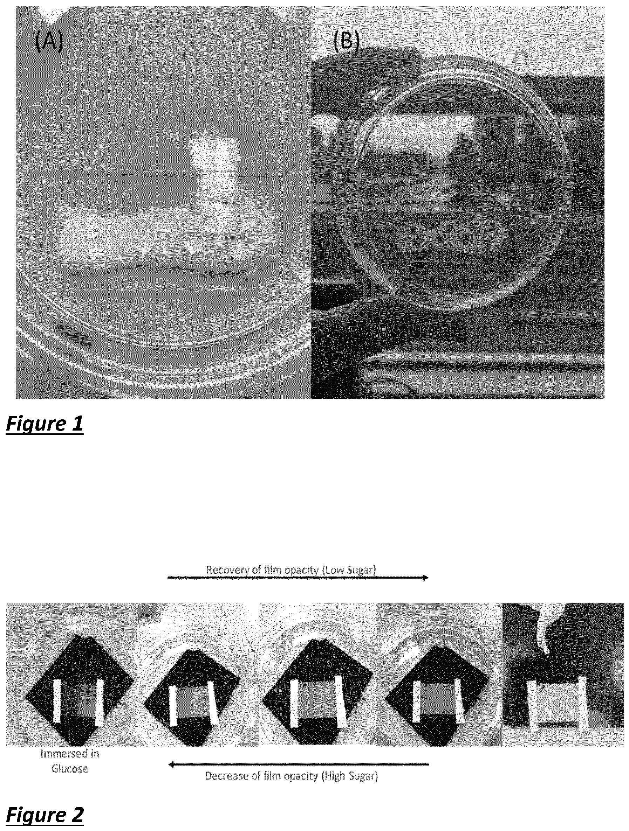

When hydrated in salt or PBS buffer solutions, the circular hydrogels or hydrogel thin films were seen to become rapidly opaque. Once the gel is brought in contact with a saccharide solution, the opacity gradually disappears, with the speed of induced transparency related to the nature and concentration of the saccharide solution. The change in opacity of the film in the presence of fructose is shown in Figure 1; Fig. 1A shows a thin film of a para-DMAPBA hydrogel after hydration in pH 7.4 PBS Buffer. On coming in contact with a saccharide solution (seen here as droplets of 100 mM fructose), the opacity of the film is quickly reduced, until the film becomes completely transparent within minutes at the locations of the droplets (Fig. 1B).

The binding of saccharides to the boronic acid receptors within the hydrogel is also observed to be reversible at physiological pH. For a thin film of hydrogel (80 µm), as shown in Figure 2, the initial transparency, induced by immersion in a 100 mM glucose solution, can be reversed over time by re-immersion in a pH 7.4 PBS solution. This clearly demonstrates that the saccharide binding, which induces a change in the morphology of the polymer chains manifested by a change in the opacity/transparency of the hydrogel film, can be reversed.

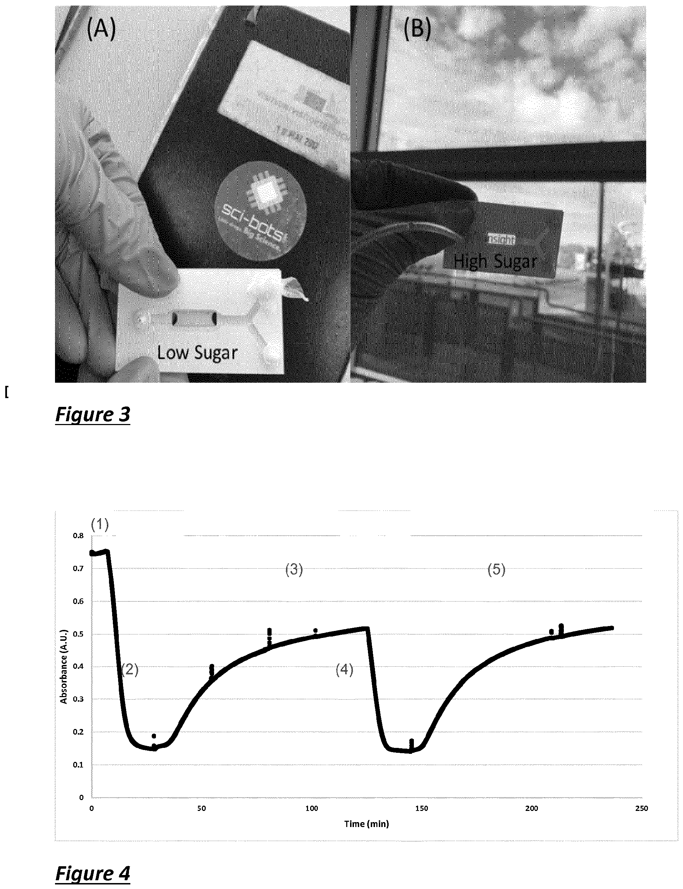

Incorporation of such a thin film into a microfluidic channel can be used to monitor real-time flow of saccharide-containing solutions, such as glucose. The optical response could be used for quantitative monitoring of glucose concentration in a wearable device, using a mobile phone camera and app or a small microprocessor. Moreover, the response, visible to the naked eye could be also used to provide a qualitative go/no-go response when glucose concentrations reach a pre-defined level of interest. Polymerisation of an 80 µm film at the bottom of a 250 µm deep Y-shaped microfluidic channel allowed the glucose binding/release responses to be observed through a small PMMA window, shown in Figure 3, in which different concentrations of saccharide could be introduced through both inlet ports.

Using a UV-vis spectrometer, the absorbance at 750 nm within the microfluidic chip was tracked. Figure 4 shows the initial high absorbance values of the opaque film in the presence of PBS buffer (1), which rapidly decreases as the film becomes increasingly transparent when 100 mM glucose solution is passed through the microfluidic chip at a flowrate of 200 µL min⁻¹. After reaching an approximate steady state (2), PBS buffer was again passed through the microchannel at a flow rate of 200 µL min⁻¹ and the absorbance increased, corresponding to the recovery of film opacity (3). A similar response to a second exposure of the film to glucose occurs (4), followed by a similar recovery in the presence of PBS buffer (5).

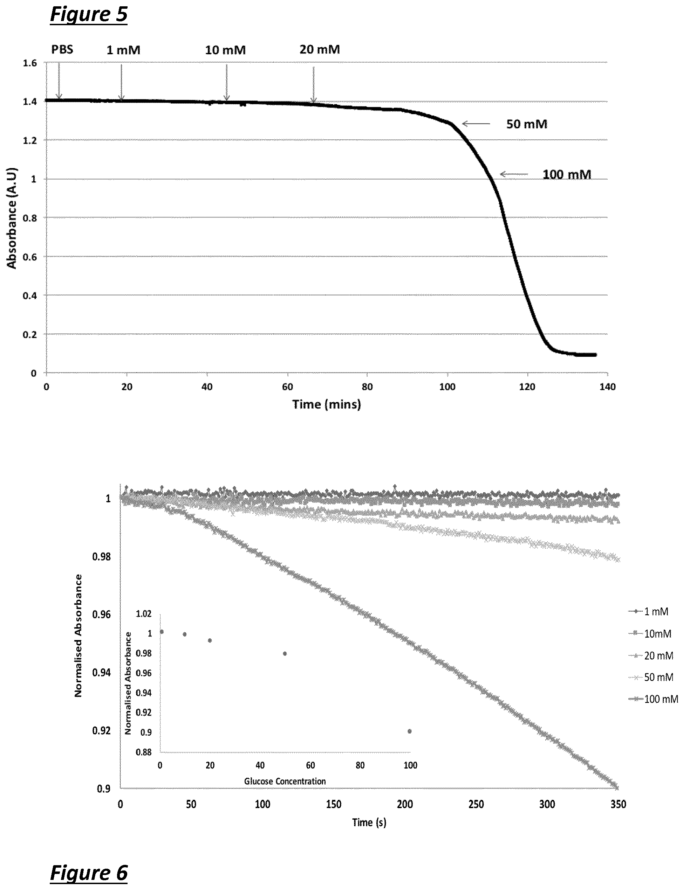

A similar experimental set-up can also be used to measure real-time changes in saccharide concentration at flowrates closer to biological relevancy (50 µL min⁻¹). Figure 5 shows the real-time recording of absorbance at 750 nm, when pH 7.4 PBS buffer and sequentially increasing glucose concentration from 1 mM-100 mM (1 mM, 10 mM, 20 mM, 50 mM and 100 mM, respectively) are passed through the micro-channel in continuous flow (50 µL min⁻¹). While exponential responses are seen in the 50 mM-100 mM range, as the gel moves to almost complete optical transparency, a large breadth of information can also be gleaned by monitoring the absolute change in optical response and behaviour of this result over time.

Figure 6 shows the response of the system for each concentration (1 mM-100 mM) after 350 seconds. A reproducible linear response in the optical change is seen for concentrations up to 20 mM. This is of considerable significance for biomedical applications, in which measured concentrations of glucose in ocular fluid and sweat range from 1-10 mM after glucose loading. The inset in Figure 6 shows the normalised change in absorbance for this time window. Indeed, from this observation, it is clear that a window as small as 100 seconds (75 µL of sample) may well be enough to perform quantitative reproducible measurements of saccharide in a sample.

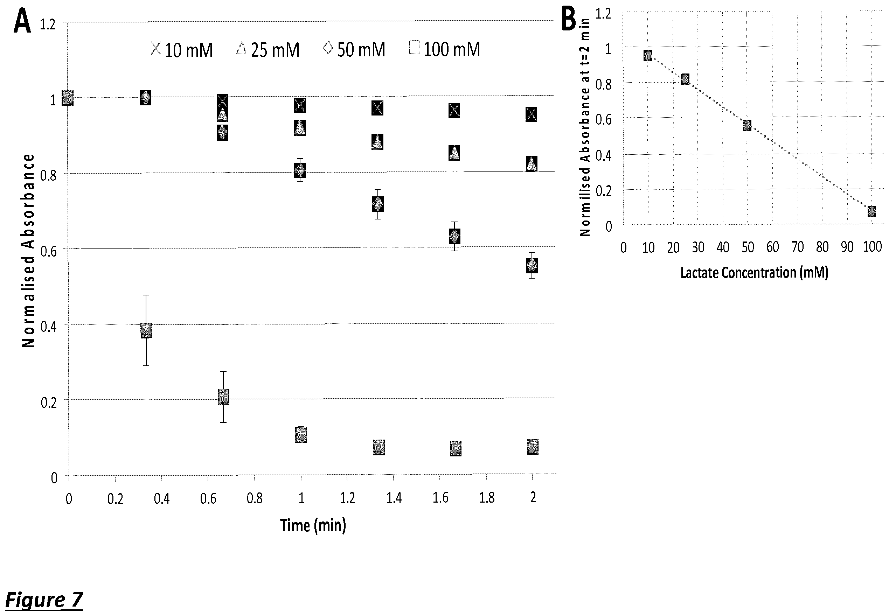

Optical response of the hydrogels to sodium lactate was carried out on a FLUOstar Omega Microplate Reader by BMG Labtech Software version 3.00 R2 and firmware version 1.32. Transparent 96-well microplates with a flat bottom were used to analyse the UV-Vis spectra of the hydrogel films in the presence of sodium lactate solutions (0-100mM). For this purpose, polymerised hydrogel thin films that have been initially hydrated in PBS buffer solution, were cut in circular disks (~5-6 mm) using a manual puncher and placed in the wells of a 96 well plate. A sodium lactate solution in PBS was added to each well, and the absorbance at 750 nm was recorded in real time (one measurement was taken every 20s). Figure 7A shows the normalised absorbance at 750 nm in real-time, in the presence of 10, 25, 50, and 100 mM sodium lactate solution in PBS, respectively. The error bars represent standard deviation from 3 different measurements. Once the hydrogel disks are brought in contact with the sodium lactate solution, the opacity (measured as absorbance at 750 nm) gradually disappears, with the speed of induced transparency related to the concentration of the sodium lactate solution. The response time of the sensor is fast. The change in opacity of the film after 2 minutes is compared for the different lactate concentrations studied (Figure 7B). The hydrogel disks are completely transparent after 2 min in the presence of 100 mM sodium lactate.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A hydrogel comprising a crosslinked polymeric matrix formed from a polymerizable boronic acid salt monomer having a general structure (II): 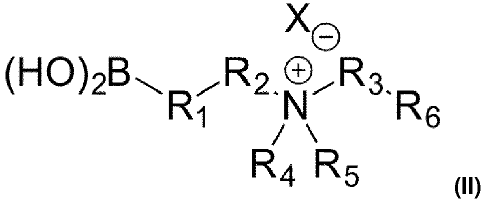 in which:
(OH)₂B-R1 is a boronic acid;
R2 to R5 are each, independently, an aryl or alkyl group;
R6 is alkyl, aryl, hydroxyl, or is a free-radical polymerizable group; and
one of X or R6 comprises a free-radical polymerizable group.

2. A hydrogel according to Claim 1 that is a sugar sensing hydrogel.

3. A hydrogel according to Claim 1 or 2, in which the polymerizable boronic acid salt has a general structure (III): 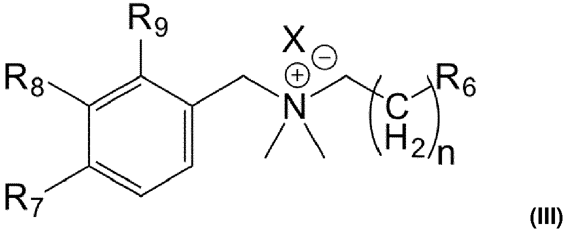
in which:R7 to R9 are each, independently, selected from B(OH)₂, H; n is a whole number selected from 0-6;
R6 is alkyl, aryl, hydroxyl, or is a free-radical polymerizable group; and one of R7 to R9 is B(OH)₂;

4. A hydrogel according to any preceding Claim, in which the boronic acid is non-fluorescent.

5. A sugar sensing hydrogel according to Claim 2 or 3, in which (OH)₂B-R1 is a phenyl boronic acid.

6. A hydrogel according to any preceding Claim, in which the anion X contains the free-radical polymerizable group, preferably, in which the anion X has a general structure (IV):
Y-(CH₂)ₘ-Z (IV)
in which:
m is a whole number selected from 0-6;
Y is a substituent selected from a sulphonate, sulphinate, phosphonate, phosphinate, and
carboxylate; and
Z is a free-radical polymerizable group typically selected from (meth)acrylamide, (meth)acrylate, styrene, vinyl ether or vinyl group.

7. A hydrogel according to any preceding Claim, in which the polymer is a homo-polymer.

8. A hydrogel according to any preceding Claim, in which the polymer matrix is a co-polymer formed from the polymerizable boronic acid salt monomer of general formula (II) and a different monomer, for example a different acrylated monomer.

9. A hydrogel according to any preceding Claim, in which the hydrogel is configured to exhibit reduced opacity on binding sugar or in which the hydrogel is configured to provide a gravimetric response on binding sugar.

10. A sensor device, the sensor device comprising a device body and a hydrogel according to any of Claims 1 to 9, associated with the device body, preferably in which the device body is selected from a plaster, patch, bandage, strap, or contact lens, or in which the device body is a microfluidic chip having at least one microfluidic channel defining a fluid path and having an opening for receipt of a test fluid and a sensing zone comprising the hydrogel in fluid communication with the fluid path.

11. A contact lens comprising a hydrogel according to any of Claims 1 to 9.

12. A polymerizable boronic acid salt having the general structure (I):
A+.X- (I)
in which:
A represents a quaternised ammonium boronic acid cation; and
X represents an anion,
wherein X contains a free-radical polymerizable group

13. A polymerizable boronic acid salt according to Claim 12, in which the polymerizable boronic acid salt has a general structure (II): 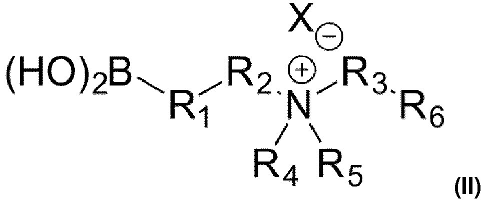 in which:
(OH)₂B-R1 is a boronic acid;
R2 to R5 are each, independently, an aryl or alkyl group; and
R6 is alkyl, aryl, or hydroxyl, and/or
in which the polymerizable boronic acid salt has a general structure (III): 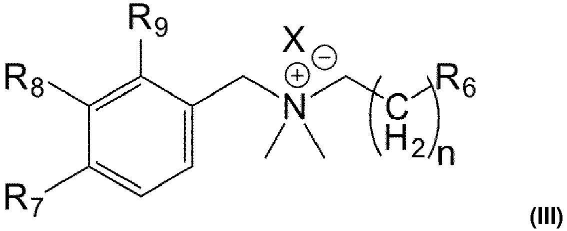 in which:
R7 to R9 are each, independently, selected from B(OH)₂, H;
n is a whole number selected from 0-6;
R6 is alkyl, aryl, or hydroxyl; and
one of R7 to R9 is B(OH)₂.

14. A polymerizable boronic acid salt according to any of Claims 12 or 13, in which the anion X has a general structure (IV):
Y-(CH₂)ₘ-Z (IV)
in which:
m is a whole number selected from 0-6;
Y is a substituent selected from a sulphonate, sulphinate, phosphonate, phosphinate, and
carboxylate; and
Z is a free-radical polymerizable group optionally selected from (meth)acrylamide, (meth)acrylate, styrene, vinyl ether or vinyl group,
preferably, in which the polymerizable boronic acid salt has the general structure (V): 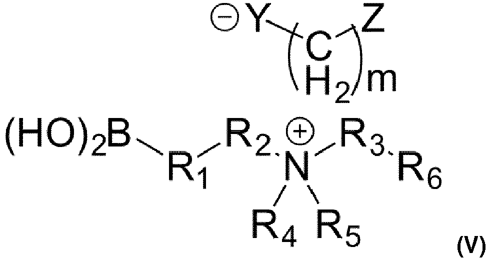 in which:
(OH)₂B-R1 is a boronic acid;
R2 to R5 are each, independently, an aryl or alkyl group; and R6 is alkyl, aryl or hydroxyl; and
Z is a free-radical polymerizable group, or
in which the polymerizable boronic acid salt has the general structure (VI): 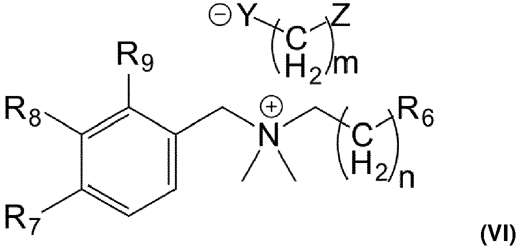 in which:
R7 to R9, Y, m, and n, are as defined above;
R6 is alkyl, aryl or hydroxyl; and
Z is a free-radical polymerizable group.

15. A method of making a hydrogel, the method comprising the steps of:
(a) pre-mixing a polymerizable boronic acid salt according to any of Claims 12 to 14 and a cross-linking agent;
(b) dissolving the pre-mixture of step (a) in water, organic solvent or a mixture of solvents;
(c) adding a radical polymerisation initiator to the pre-mixture to form a mixture; and
(d) polymerizing the mixture, preferably in which the radical polymerisation initiator is a UV light initiator, and in which the method includes a step of exposing the mixture to UV light.

16. A curable composition comprising a polymerizable boronic acid according to any of Claims 12 to 14, a cross-linking agent, and a radical polymerisation initiator.

## Patentansprüche

1. Hydrogel, das eine vernetzte Polymermatrix umfasst, die aus einem polymerisierbaren Boronsäuresalz-Monomer gebildet ist, das eine allgemeine Struktur (II) aufweist: 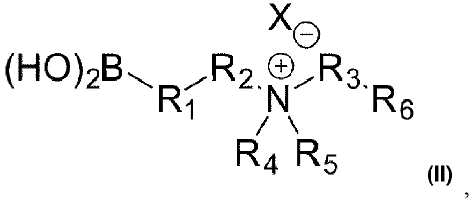 in der:
(OH)₂B-R1 eine Boronsäure ist;
R2 bis R5 jeweils unabhängig eine Aryl- oder Alkylgruppe sind;
R6 Alkyl, Aryl, Hydroxyl ist oder eine radikalische polymerisierbare Gruppe ist; und
eines von X oder R6 eine radikalische polymerisierbare Gruppe umfasst.

2. Hydrogel nach Anspruch 1, das ein Zucker-sensierendes Hydrogel ist.

3. Hydrogel nach Anspruch 1 oder 2, in dem das polymerisierbare Boronsäuresalz eine allgemeine Struktur (III) aufweist: 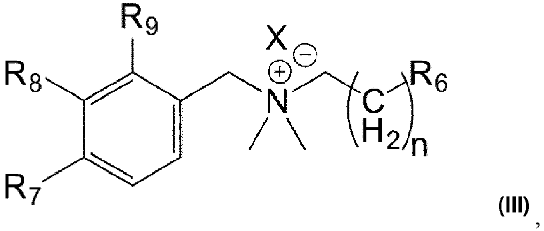
in der: R7 bis R9 jeweils unabhängig aus B(OH)₂, H ausgewählt sind;
n eine ganze Zahl ist, die aus 0-6 ausgewählt ist;
R6 Alkyl, Aryl, Hydroxyl ist oder eine radikalische polymerisierbare Gruppe ist; und eines von R7 bis R9 B(OH)₂ ist.

4. Hydrogel nach einem vorstehenden Anspruch, in dem die Boronsäure nicht fluoreszierend ist.

5. Zucker-sensierendes Hydrogel nach Anspruch 2 oder 3, in dem (OH)₂B-R1 eine Phenylboronsäure ist.

6. Hydrogel nach einem vorstehenden Anspruch, in dem das Anion X die radikalische polymerisierbare Gruppe enthält, bevorzugt in dem das Anion X eine allgemeine Struktur (IV) aufweist:
Y-(CH₂)ₘ-Z (IV)
in der:
m eine ganze Zahl ist, die aus 0-6 ausgewählt ist;
Y ein Substituent ist, der aus einem Sulfonat, Sulfinat, Phosphonat, Phosphinat und
Carboxylat ausgewählt ist; und
Z eine radikalische polymerisierbare Gruppe ist, die typischerweise aus (Meth)acrylamid-, (Meth)acrylat-, Styrol-, Vinylether- oder Vinylgruppe ausgewählt ist.

7. Hydrogel nach einem vorstehenden Anspruch, in dem das Polymer ein Homopolymer ist.

8. Hydrogel nach einem vorstehenden Anspruch, in dem die Polymermatrix ein Copolymer ist, das aus dem polymerisierbaren Boronsäuresalz-Monomer der allgemeinen Formel (II) und einem anderen Monomer, beispielsweise einem anderen acrylierten Monomer, gebildet ist.

9. Hydrogel nach einem vorstehenden Anspruch, in dem das Hydrogel konfiguriert ist, um eine verringerte Opazität beim Binden von Zucker zu zeigen, oder in dem das Hydrogel konfiguriert ist, um eine gravimetrische Reaktion beim Binden von Zucker bereitzustellen.

10. Sensorvorrichtung, wobei die Sensorvorrichtung einen Vorrichtungskörper und ein Hydrogel nach einem der Ansprüche 1 bis 9, der mit dem Vorrichtungskörper in Verbindung ist, umfasst, bevorzugt in der der Vorrichtungskörper aus einem Pflaster, Patch, Verband, Band oder einer Kontaktlinse ausgewählt ist oder in der der Vorrichtungskörper ein mikrofluidischer Chip ist, der mindestens einen mikrofluidischen Kanal aufweist, der einen Flüssigkeitspfad definiert, und eine Öffnung zur Aufnahme einer Testflüssigkeit und eine Sensorzone, die das Hydrogel in Flüssigkeitsverbindung mit dem Flüssigkeitspfad umfasst, aufweist.

11. Kontaktlinse, die ein Hydrogel nach einem der Ansprüche 1 bis 9 umfasst.

12. Polymerisierbares Boronsäuresalz, das die allgemeine Struktur (I) aufweist:
A+.X- (I)
in der:
A ein quartäres Ammoniumboronsäure-Kation darstellt; und
X ein Anion darstellt,
wobei X eine radikalische polymerisierbare Gruppe enthält.

13. Polymerisierbares Boronsäuresalz nach Anspruch 12, in dem das polymerisierbare Boronsäuresalz eine allgemeine Struktur (II) aufweist: 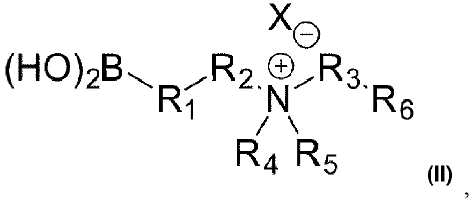 in der:
(OH)₂B-R1 eine Boronsäure ist;
R2 bis R5 jeweils unabhängig eine Aryl- oder Alkylgruppe sind; und
R6 Alkyl, Aryl oder Hydroxyl ist, und/oder
in dem das polymerisierbare Boronsäuresalz eine allgemeine Struktur (III) aufweist: 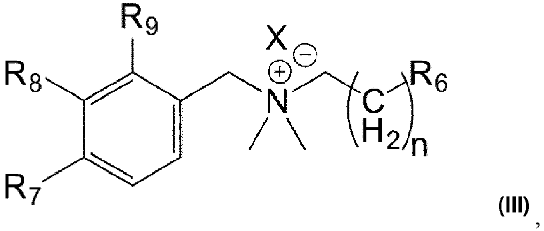 in der:
R7 bis R9 jeweils unabhängig aus B(OH)₂, H ausgewählt sind;
n eine ganze Zahl ist, die aus 0-6 ausgewählt ist;
R6 Alkyl, Aryl oder Hydroxyl ist; und
eines von R7 bis R9 B(OH)₂ ist.

14. Polymerisierbares Boronsäuresalz nach einem der Ansprüche 12 oder 13, in dem das Anion X eine allgemeine Struktur (IV) aufweist:
Y-(CH₂)ₘ-Z (IV)
in der:
m eine ganze Zahl ist, die aus 0-6 ausgewählt ist;
Y ein Substituent ist, der aus einem Sulfonat, Sulfinat, Phosphonat, Phosphinat und
Carboxylat ausgewählt ist; und
Z eine radikalische polymerisierbare Gruppe ist, die optional aus (Meth)acrylamid-, (Meth)acrylat-, Styrol-, Vinylether- oder Vinylgruppe ausgewählt ist,
bevorzugt in dem das polymerisierbare Boronsäuresalz die allgemeine Struktur (V) aufweist: 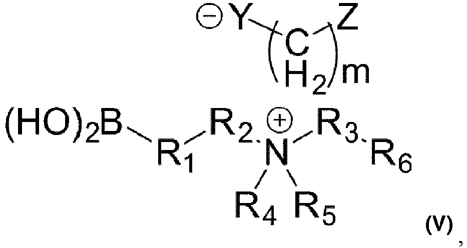 in der:
(OH)₂B-R1 eine Boronsäure ist;
R2 bis R5 jeweils unabhängig eine Aryl- oder Alkylgruppe sind; und R6 Alkyl, Aryl oder Hydroxyl ist; und
Z eine radikalische polymerisierbare Gruppe ist, oder
in dem das polymerisierbare Boronsäuresalz die allgemeine Struktur (VI) aufweist: 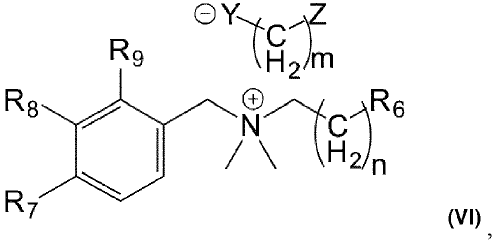 in der:
R7 bis R9, Y, m und n wie vorstehend definiert sind;
R6 Alkyl, Aryl oder Hydroxyl ist; und
Z eine radikalische polymerisierbare Gruppe ist.

15. Verfahren zur Herstellung eines Hydrogels, wobei das Verfahren die folgenden Schritte umfasst:
(a) Vormischen eines polymerisierbaren Boronsäuresalzes nach einem der Ansprüche 12 bis 14 und eines Vernetzungsmittels;
(b) Auflösen der Vormischung von Schritt (a) in Wasser, organischem Lösungmittel oder einer Mischung aus Lösungsmitteln;
(c) Zugeben eines Starters der radikalischen Polymerisation zu der Vormischung, um eine Mischung zu bilden; und
(d) Polymerisieren der Mischung, bevorzugt in dem der Starter der radikalischen Polymerisation ein UV-Licht-Starter ist und in dem das Verfahren einen Schritt des Aussetzens der Mischung gegenüber UV-Licht einschließt.

16. Härtbare Zusammensetzung, die eine polymerisierbare Boronsäure nach einem der Ansprüche 12 bis 14, ein Vernetzungsmittel und einen Starter der radikalischen Polymerisation umfasst.

## Revendications

1. Hydrogel comprenant une matrice polymère réticulée, formée à partir d'un monomère de sel d'acide boronique polymérisable répondant à la structure générale (II) : 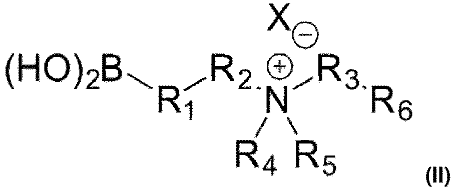 dans laquelle :
(OH)₂B-R1 est un acide boronique ;
R2 à R5 sont chacun, indépendamment, un groupement aryle ou alkyle ;
R6 est alkyle, aryle, hydroxyle, ou est un groupement polymérisable par voie radicalaire ; et
l'un parmi X ou R6 comprend un groupement polymérisable par voie radicalaire.

2. Hydrogel selon la revendication 1, qui est un hydrogel détecteur de sucre.

3. Hydrogel selon la revendication 1 ou 2, dans lequel le sel d'acide boronique polymérisable répond à la structure générale (III) : 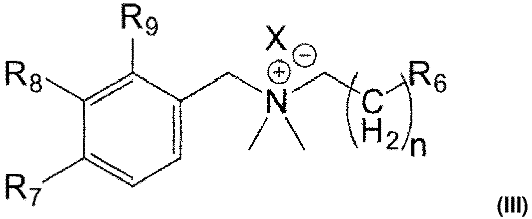 dans laquelle :
R7 à R9 sont choisis chacun indépendamment parmi B(OH)₂, H ;
n est un nombre entier choisi parmi 0-6 ;
R6 est alkyle, aryle, hydroxyle, ou est un groupement polymérisable par voie radicalaire ; et
l'un parmi R7 à R9 est B(OH)₂.

4. Hydrogel selon l'une quelconque des revendications précédentes, dans lequel l'acide boronique est non fluorescent.

5. Hydrogel détecteur de sucre selon la revendication 2 ou 3, dans lequel (OH)₂B-R1 est un acide phénylboronique.

6. Hydrogel selon l'une quelconque des revendications précédentes, dans lequel l'anion X contient le groupement polymérisable par voie radicalaire, préférablement, où l'anion X répond à la structure générale (IV) :
Y-(CH₂)ₘ-Z (IV)
dans laquelle :
m est un nombre entier choisi parmi 0-6 ;
Y est un substituant sélectionné parmi un sulfonate, un sulfinate, un phosphonate, un phosphinate et un carboxylate ; et
Z est un groupement polymérisable par voie radicalaire choisi typiquement parmi un groupement (méth)acrylamide, (méth)acrylate, styrène, éther de vinyle ou vinyle.

7. Hydrogel selon l'une quelconque des revendications précédentes, dans lequel le polymère est un homopolymère.

8. Hydrogel selon l'une quelconque des revendications précédentes, dans lequel la matrice polymère est un copolymère formé à partir du monomère de sel d'acide boronique polymérisable de formule générale (II) et d'un monomère différent, par exemple un monomère acrylé différent.

9. Hydrogel selon l'une quelconque des revendications précédentes, l'hydrogel étant configuré pour présenter une opacité réduite lors de la fixation de sucre ou l'hydrogel étant configuré pour fournir une réponse gravimétrique lors de la fixation de sucre.

10. Dispositif de détection, le dispositif de détection comprenant un corps de dispositif et un hydrogel selon l'une quelconque des revendications 1 à 9, associé au corps de dispositif, préférablement où le corps de dispositif est choisi parmi un plâtre, un patch, un bandage, une bande ou une lentille de contact, ou où le corps de dispositif est une puce microfluidique ayant au moins un canal microfluidique définissant un chemin de fluide et ayant une ouverture pour recevoir un fluide à tester et une zone de détection comprenant l'hydrogel en communication fluidique avec le chemin de fluide.

11. Lentille de contact, comprenant un hydrogel selon l'une quelconque des revendications 1 à 9.

12. Sel d'acide boronique polymérisable, répondant à la formule générale (I) :
A+.X- **(I)**
dans laquelle :
A représente un cation d'acide boronique d'ammonium quaternisé ; et
X représente un anion ;
où X contient un groupement polymérisable par voie radicalaire.

13. Sel d'acide boronique polymérisable selon la revendication 12, le sel d'acide boronique polymérisable répondant à la structure générale (II) : 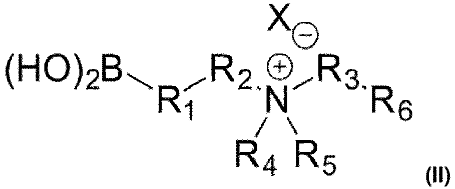 dans laquelle :
(OH)₂B-R1 est un acide boronique ;
R2 à R5 sont chacun, indépendamment, un groupement aryle ou alkyle ; et
R6 est alkyle, aryle, ou hydroxyle, et/ou
le sel d'acide boronique polymérisable répondant à la structure générale (III) : 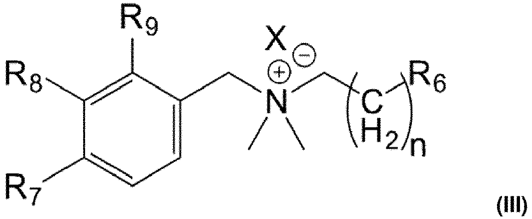
dans laquelle :
R7 à R9 sont choisis chacun indépendamment parmi B(OH)₂, H ;
n est un nombre entier choisi parmi 0-6 ;
R6 est alkyle, aryle, ou hydroxyle ; et
l'un parmi R7 à R9 est B(OH)₂.

14. Sel d'acide boronique polymérisable selon l'une quelconque des revendications 12 ou 13, dans lequel l'anion X répond à la structure générale (IV) :
Y-(CH₂)ₘ-Z **(IV)**
dans laquelle :
m est un nombre entier choisi parmi 0-6 ;
Y est un substituant sélectionné parmi un sulfonate, un sulfinate, un phosphonate, un phosphinate et un carboxylate ; et
Z est un groupement polymérisable par voie radicalaire choisi éventuellement parmi un groupement (méth)acrylamide, (méth)acrylate, styrène, éther de vinyle ou vinyle ;
préférablement, le sel d'acide boronique polymérisable répondant à la structure générale (V) : 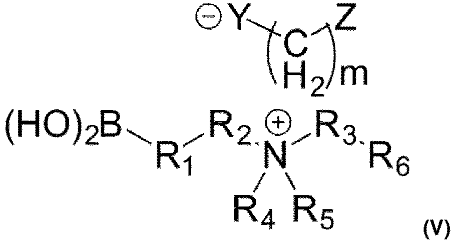 dans laquelle :
(OH)₂B-R1 est un acide boronique ;
R2 à R5 sont chacun, indépendamment, un groupement aryle ou alkyle ; et R6 est alkyle, aryle, ou hydroxyle ; et
Z est un groupement polymérisable par voie radicalaire ; ou
le sel d'acide boronique polymérisable répondant à la structure générale (VI) : 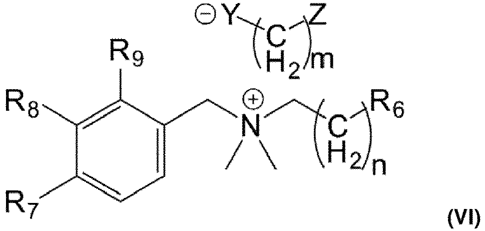
dans laquelle :
R7 à R9, Y, m et n sont tels que définis ci-dessus ;
R6 est alkyle, aryle, ou hydroxyle ; et
Z est un groupement polymérisable par voie radicalaire.

15. Méthode de préparation d'un hydrogel, la méthode comprenant les étapes :
(a) de prémélange d'un sel d'acide boronique polymérisable selon l'une quelconque des revendications 12 à 14 et d'un agent de réticulation ;
(b) de solubilisation du prémélange de l'étape (a) dans de l'eau, un solvant organique ou un mélange de solvants ;
(c) d'addition d'un initiateur de polymérisation radicalaire au prémélange afin de former un mélange ; et
(d) de polymérisation du mélange, préférablement où l'initiateur de polymérisation radicalaire est un photo-initiateur UV, et la méthode comprenant une étape d'exposition du mélange à la lumière UV.

16. Composition durcissable, comprenant un acide boronique polymérisable selon l'une quelconque des revendications 12 à 14, un agent de réticulation, et un initiateur de polymérisation radicalaire.
